(19) Europäisches Patentamt
European Patent Office
Office européen des brevets

(11) EP 2 386 353 B1

(12) # EUROPEAN PATENT SPECIFICATION

(45) Date of publication and mention of the grant of the patent:
**24.07.2013 Bulletin 2013/30**

(51) Int Cl.:
***B01J 13/18*** *(2006.01)* ***B01J 13/00*** *(2006.01)*

(21) Application number: **11171210.5**

(22) Date of filing: **10.09.2009**

(54) **Suspensions of silicate shell microcapsules**

Suspensionen von Mikrokapseln mit Silikathülle

Suspensions de microcapsules de coque en silicate

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.09.2008 US 96397 P**

(43) Date of publication of application:
**16.11.2011 Bulletin 2011/46**

(62) Document number(s) of the earlier application(s) in accordance with Art. 76 EPC:
**09792407.0 / 2 326 413**

(73) Proprietor: **Dow Corning Corporation**
**Midland, MI 48686-0994 (US)**

(72) Inventors:
- **Galeone, Fabrizio, S.**
  **B-7133 Buvrinnes (BE)**
- **Marteaux, Leon, Andre**
  **B-1160 Bruxelles (Auderghem) (BE)**
- **Zimmerman, Brett**
  **Frankenumth, MI Michigan 48734 (US)**

(74) Representative: **Thomson, Craig Richard**
**Murgitroyd & Company**
**Scotland House**
**165-169 Scotland Street**
**Glasgow G5 8PL (GB)**

(56) References cited:
**EP-A2- 0 934 773**
**WO-A1-2005/009604**
**WO-A2-00/09652**
**WO-A2-00/72806**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 386 353 B1

## Description

### Cross Reference to Related Applications

This application claims the benefit of US Patent Application No. 61/096397 as filed on September 12, 2008.

### *Technical Field*

This disclosure relates to a processes for obtaining an aqueous suspensions of silicate shell microcapsules having a volume fraction of microcapsules of at least 30 % that are gel free at 50°C for at least one month.

### Background

Suspensions of microcapsules having a silicate shell and a liquid core are known. Representative examples of these types of core-shell microcapsules suspensions are described in EP 0934773, EP 0941761, US 6,303,149, WO 03/066209, and GB 2416524.

WO 2005/009604 A1 discloses a process for preparing an aqueous suspension of microcapsules comprising a silica shell, said aqueous suspension being substantially free of colloidal silica.

A significant problem with current lipophilic liquid core / silicate shell microcapsule suspensions are their tendency to gel under certain conditions. For example, gelling often occurs with an increase in the microcapsule size or at higher volume fractions of the microcapsules in the suspension. Also, gelling may occur when the temperature of the suspension increases, or with pH changes. However, the cause of the gelling is uncertain, and there remains a need to provide suspensions of microcapsules having improved stability.

The present inventors believe suspensions of such microcapsules gel and have limited stability because free nanoparticles of colloidal silica are present in the suspension compositions. The inventors believe these colloidal silica particles may aggregate to form a gel with time, especially at elevated temperatures and/or non-neutral pH conditions. Accordingly, we have discovered that if the concentration of the free nanoparticles of colloidal silica in the continuous phase of the suspension is either reduced, or rendered inactive, gelation may be reduced or eliminated. Thus the present disclosure relates to suspensions of microcapsules composition with improved stability, although such suspensions do not form part of the claimed invention. The disclosed processes also provide microcapsule suspensions with improved stability having a higher volume fraction of microcapsules. The present process also provides microcapsule suspensions with improved thermal and pH stability of the suspension. The present process yet further provides compositions characterized in that the shell thickness of the lipophilic liquid core / silicate shell microcapsules are significantly enhanced without gelation.

### Summary

This disclosure relates to aqueous suspensions of silicate shell microcapsules having a volume fraction of microcapsules of at least 30 % and are gel free at 50°C for at least one month, although such suspensions do not form part of the claimed invention.

This disclosure further relates to a process for improving the stability of an aqueous suspension of silicate shell microcapsules comprising reducing non-volatile solid content of the continuous phase of the aqueous suspension of the silicate shell microcapsules to less than 0.3 weight percent.

This disclosure further relates to a process for improving the stability of an aqueous suspension of silicate shell microcapsules comprising adding a colloidal silicate sequestering agent to an aqueous suspension of silicate shell microcapsules and colloidal silicate particles, although such a process does not form part of the claimed invention.

### Detailed Description

Silicate shell microcapsules are typically produced using aqueous suspension techniques involving the polymerization of silicate precursors such as a tetraalkoxysilane. The resulting silicate shell microcapsule suspension compositions often have limited storage stabilities, especially with regard to temperature and pH changes.

While not wishing to be bound by any theory, the present inventors believe the presence of colloidal silicate particles in the suspension of silicate shell microcapsules may be a major cause of the storage instability of these suspensions. Such colloid silicate particles may be considered as a side product in the tetraalkoxysilane polymerization reaction to produce the silicate shell microcapsule. The present inventors have discovered that the storage stability of suspensions of silicate shell microcapsules is improved when the amount of colloidal silicate particles in the suspension is reduced, or alternatively, are rendered non-reactive by the addition of a sequestering agent.

The present disclosure provides aqueous suspensions of silicate shell microcapsules having improved storage stability, although such suspensions do not form part of the claimed invention. The disclosed aqueous suspensions of silicate shell microcapsules have a volume fraction of at least 30 % and are gel free at 50°C for at least one month.

As used herein "volume fraction" refers to the amount of the silicate shell microcapsules in the suspension on a volume/volume basis. Volume fraction can be calculated by summing the volume occupied by each component added to the suspension, as determined by dividing the mass used of each component by its density. The suspensions of silicate shell microcapsules have at least 30 %, alternatively, 40%, or alternatively, 50% volume fraction of the microcapsules in suspension.

As used herein "gel free" means the viscosity of the suspension does not significantly increase with time. This may be assessed by simple visual comparisons of the starting vs aged suspensions. Typically, the suspensions have relatively low viscosity since they are aqueous based.
If upon aging at elevated temperatures, the suspension behaves like a gel, this indicates a lack of stability. For purposes of this disclosure, a suspension is considered to be gelled if it does not flow at room temperature.

The inventors believe colloidal silicate particles may account for a major portion of the non-volatile content of the continuous phase of the aqueous suspension of the silicate shell microcapsules. The present inventors have found that reducing the non-volatile content of the continuous phase of the suspension improves storage stability. As used herein, the "suspension" is defined to contain a dispersed phase of the solid silicate shell microcapsules in an aqueous continuous phase. Typically, the solid silicate shell microcapsules range in size from 1 to 5 micrometers. Other components and materials, not contained in the silicate shell microcapsule, are considered to be part of the "continuous phase" of the suspension. Thus, in one embodiment, the present process involves reducing the non-volatile solid content of the continuous phase of the suspension of the silicate shell microcapsules to less than 0.3 weight percent, alternatively to less than 0.2 weight percent, or alternatively to less than 0.1 weight percent. As used herein, "the non-volatile solid content" refers to the mass of solid material remaining after the continuous phase of the suspension (as separated from the microcapsules) is subjected to conditions to allow water and other volatile materials in the composition to evaporate. For purposes of this disclosure, those conditions are defined as subjecting 2.5 g of the composition in an open container placed in an oven at 170°C until a constant weight is achieved (constant weight is defined as a weight change of less than 1% for 2 hours).

In another embodiment, the non volatile solid content of the continuous phase of the suspension is reduced by removing at least 50 weight %, alternatively 70 weight %, alternatively 90 weight % of the colloidal silicate particles from the continuous phase of the aqueous suspension of the silicate shell microcapsules.

The colloidal silicate particles may be removed from the suspension of silicate shell microcapsules by any technique or process. The colloidal silicate particles may be considered as "nanoparticles" having sizes that average less than 400 nanometers. In one embodiment, the colloidal silicate particles are removed from the continuous phase of the aqueous suspension of the silicate shell microcapsules by ultrafiltration. As used herein, "ultrafiltration" refers to a separation process whereby the suspension of silicate shell microcapsules containing colloidal silicate particles is subjected to a hydraulic pressure which forces water and the colloidal silicate particles through a suitable membrane. Thus, the colloidal silicate particles are removed from the suspension of silicate shell microcapsules during the ultrafiltration process, and collected as permeate in the ultrafiltration process. Typically, the membrane has a pore size of 500 nm, alternatively 450 nm, or alternatively 400 nm. Representative examples of suitable membranes include those available from Millipore (Billerica, MA USA). Typically, a hydraulic pressure of at least 1.4 MPa (200 psi) or 1.2 MPa is applied to the suspension during the ultrafiltration process. Representative ultrafiltration lab equipment are those provided by Millipore (Billerica, MA USA). Representative ultrafiltration manufacturing equipment are those provided by Tami Industries (TAMI Industries Nyons France) or Pall Corporation (East Hills, NY 11548).

In a further embodiment, the ultrafiltration occurs with parallel addition of water to the suspension of the silicate shell microcapsules. As used herein, "parallel addition of water" means simultaneously adding sufficient amounts of water to the suspension of the silicate shell microcapsule suspension during the ultrafiltration process to replace the amount of water removed as the permeate of the ultrafiltration process. Typically, the amount of water added to the suspension may vary from 90 to 110% of the water removed in the ultrafiltration process as permeate.

As an alternative approach to removing the colloidal silica particles, the present inventors believe the stability of aqueous suspensions of silicate shell microcapsules may be improved by preventing coagulation and/or reaction of the silica particles together. Thus, although not forming part of the presently claimed invention, the present disclosure also provides a process for improving the stability of an aqueous suspension of silicate shell microcapsules comprising adding a colloidal silicate sequestering agent to an aqueous suspension of silicate shell microcapsules and colloidal silicate particles. As used herein "a colloidal silicate sequestering agent" refers to any compound or material that when added to the silicate shell microcapsule suspension which also contains colloid silica particles, interacts with the colloidal silicate particles in such a manner so as to prevent their reaction or coagulation.

The colloidal silicate sequestering agent may be an organofunctional silane. The organofunctional silane is a quat functional trialkoxysilane. Representative, non-limiting examples of suitable quat functional trialkoxysilanes include

Dow Corning ® Q9-6346 - Cetrimoniumpropyltrimethoxysilane Chloride.

The colloidal silicate sequestering agent may be a silicone polyether. The silicone polyether may be selected from those having the structure represented by:

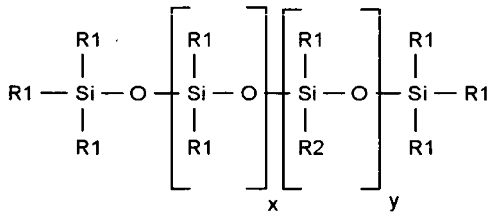

or

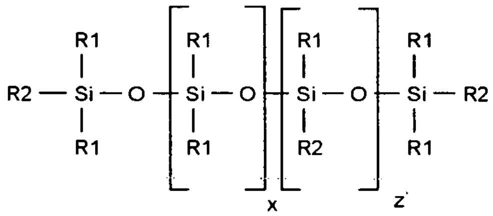

In these structures, R1 represents an alkyl group containing 1-6 carbon atoms such as methyl, ethyl, propyl, butyl, pentyl, and hexyl; R2 represents a polyether group having the formula $-(CH_2)_aO(C_2H_4O)_b(C_3H_6O)_cR3$; x has a value of 10-1,000, alternatively 20-200, or alternatively 20-100; y has a value of 2-500, alternatively 2-50, or alternatively 2-10, z has a value of 2-500, alternatively 2-50, or alternatively 2-10; a has a value of 3-6; b has a value of 4-30; c has a value of 0-30; and R3 is hydrogen, a methyl radical, or an acyl radical such as acetyl. Typically, R1 is methyl. Silicone polyethers are commercially available. Representative, non-limiting examples of suitable silicone polyethers include Dow Corning ® 190, 193, and 2-5657.

The suspensions of silicate shell microcapsules may be prepared by any process known in the art. In general, there are two processes or techniques commonly used to prepare silicate shell microcapsules. The first technique involves an in-situ (sometimes referred to as a sol-gel process) polymerization of a silicate precursor, after first mixing the silicate precursor with an oil phase.

Representative, non limiting examples are those taught in US 6159453, US 6238650, US 6303149, and WO 2005/009604.

The second technique involves an ex-situ process, where the polymerization of a silicate precursor occurs via an emulsion polymerization process. Representative, non-limiting examples of such techniques are taught in WO03/066209.

In one embodiment, the silicate shell microcapsules are prepared by;

I) mixing an oil phase and an aqueous solution of a cationic surfactant to form an oil in water emulsion,

II) adding a water reactive silicon compound comprising a tetraalkoxysilane to the oil in water emulsion,

III) polymerizing the tetraalkoxysilane at the oil/water interface of the emulsion to form a microcapsule having a core containing the oil and a silicate shell.

As used herein, "oil phase" encompasses any compound, or mixture of compounds that is hydrophobic. Typically, the oil phase is liquid when forming the oil in water emulsion. The oil phase may contain any organic, silicone, or fluorocarbon based oil, either alone or in combination. The oil phase may also contain any solvent or diluent, which may be added for the purpose of solubilizing solid hydrophobic compounds to create a liquid oil phase during formation of the emulsion.

In one embodiment, the oil phase contains a sunscreen agent. The sunscreen agents which are used in this embodiment can be liquid sunscreens and blends thereof. In the same embodiment of this invention solid organic sunscreens can be solubilised in a good solvent before encapsulation. Sunscreen agents in this invention might be for example DEA-methoxycinnamate, diethylhexylbutamido triazine,diisopropyl methyl cinnamate, 1-(3,4-dimethoxyphenyl)-4,4-dimethyl-1,3-pentanedione, drometrizole trisiloxane, benzophenone-3, benzophenone-4, 3-benzylidene camphor, 3-benzylidene camphor sulfonic acid, bis-ethylhexyloxyphenol methoxyphenyl triazine, butyl methoxydibenzoylmethane, camphor benzalkonium methosulfate, ethyl diisopropylcinnamate, 2-ethylhexyl dimethoxybenzylidene dioxoimidazolidine propionate, ethylhexyl dimethyl PABA, ethylhexyl salicilate, ethylhexyl triazone, ethyl PABA, homosalate, isoamyl p-methoxycinnamate, menthyl anthranilate, 4-methylbenzylidene camphor, methylene-bis-benzotriazolyl tetramethylbutylphenol, octocrylene, PABA, phenylbenzimidazole sulfonic acid, polyacrylamidomethyl benzylidene camphor, polysilicone-15, potassium phenylbenzimidazole sulfonate, sodium phenylbenzimidazole sulfonate, TEA-salicilate, terephtalidene dicamphor sulfonic acid, 2,2-(1,4-phenilene)bis-(1 H-benzimidazole-4,6-disulfonic acid, 2-(4-diethylamino-2-hydroxy-benzoyl)-benzoic acid hexylester but is not limited to this list of UV absorber.

Other examples of active materials which may be used in the oil phase of the present process include UV absorbers used in coatings, paints, plastics materials, sealants or textile finishes for improving weatherability and resisting fading.

The oil phase may contain other components such as a silicone, organic, or personal care actives that are substantially soluble with the other oil phase components, and conversely, is substantially insoluble in water. Thus, other typical emollient components can include: silicones, such as volatile siloxanes, polydimethylsiloxane fluids, high molecular weight (i.e. $M_w > 1000$) siloxanes, including silicone elastomers and resins; organic compounds such as, hydrocarbon oils, waxes, emollients, fragrances or perfume compositions; and personal care organic actives such as vitamins.

The oil phase is mixed with an aqueous solution of a cationic surfactant to form an oil in water emulsion.

Cationic surfactants useful in this invention might be quaternary ammonium hydroxides such as octyl trimethyl ammonium hydroxide, dodecyl trimethyl ammonium hydroxide, hexadecyl trimethyl ammonium hydroxide, octyl dimethyl benzyl ammonium hydroxide, decyl dimethyl benzyl ammonium hydroxide, didodecyl dimethyl ammonium hydroxide, dioctadecyl dimethyl ammonium hydroxide, tallow trimethyl ammonium hydroxide and coco trimethyl ammonium hydroxide as well as corresponding salts of these materials, fatty amines and fatty acid amides and their derivatives, basic pyridinium compounds, quaternary ammonium bases of benzimidazolines and polypropanolpolyethanol amines but is not limited to this list of cationic surfactants. A preferred cationic surfactant is cetyl trimethyl ammonium chloride.

For purposes of this invention, the cationic surfactant may be selected from an amphoteric surfactant such as cocamidopropyl betaine, cocamidopropyl hydroxysulfate, cocobetaine, sodium cocoamidoacetate, cocodimethyl betaine, N-coco-3-aminobutyric acid and imidazolinium carboxyl compounds but is not limited to this list of amphoteric surfactants.

The above surfactants may be used individually or in combination. The cationic or amphoteric surfactant is dissolved in water and the resulting aqueous solution used as a component in aqueous or continuous phase of the oil in water emulsion of step 1).

Although not wishing to be bound by any theory, the present inventors believe the use of a cationic or amphoteric surfactant promotes condensation and polymerisation of the tetraalkoxysilane at the interface of the emulsified droplets of the sunscreen agent composition, leading to non-diffusive microcapsules. The tetraalkoxysilane hydrolyzes and condenses upon reacting in the emulsion. The anionically charged hydrolysis product is attracted to the cationic or amphoteric surfactant at the interface where it forms the silicon based polymer shell.

The concentration of the cationic surfactant during the formation of the oil in water emulsion should be between 0.1 % and 0.3% by weight of the oil phase concentration used. We have found that the use of low levels of cationic or amphoteric surfactant during emulsification of the oil phase and reaction with the alkoxysilane leads to microcapsules which are more resistant to diffusion or leaching of the oil phase from the microcapsules.

Auxiliary surfactants, and in particular nonionic surfactants, may be added during the formation of the oil in water emulsion. Suitable non-ionic surfactants are; polyoxyalkylene alkyl ethers such as polyethylene glycol long chain (12-14C) alkyl ether, polyoxyalkylene sorbitan ethers, polyoxyalkylene alkoxylate esters, polyoxyalkylene alkylphenol ethers, ethylene glycol propylene glycol copolymers, polyvinyl alcohol and alkylpolysaccharides, for example materials of the structure $R^1$-O-($R^2$O)$_m$-(G)$_n$ wherein $R^1$ represents a linear or branched alkyl group, a linear or branched alkenyl group or an alkylphenyl group, $R^2$ represent an alkylene group, G represents a reduced sugar, m denotes 0 or a positive integer and n represent a positive integer as described in US Patent 5,035,832 but is not limited to this list of non-ionic surfactants.

The aqueous solution of the cationic or amphoteric surfactant may contain additional/optional components, providing they are water soluble. For example a water-miscible organic solvent such as an alcohol or lactam may be added. Furthermore, other water soluble ingredients that are commonly used in personal care formulations may be added to the aqueous phase. Such ingredients include additional surfactants, thickeners, preservatives, antimicrobial, and water soluble actives and fragrances.

The oil phase and aqueous solution of the cationic or amphoteric surfactant are mixed together to form an oil in water emulsion. Mixing and emulsion formation may occur using any known techniques in the emulsion art. Typically, the oil phase and aqueous solution of the cationic or amphoteric surfactant are combined using simple stirring techniques to form an emulsion. Particle size of the oil in water emulsion may then be reduced before addition of the tetraalkoxysilane by any of the known in the art emulsification device. Useful emulsification devices in this invention can be homogenizer, sonolator, rotor-stator turbines, colloid mill, microfluidizer, blades, helix and combination thereof but is not limited to this

list of emulsification devices. This further processing step reduces the particle size of the starting cationic oil in water emulsion to values ranging from 0.2 to 500 micrometers, with typical particle sizes ranging between 0.5 micrometers and 100 micrometers.

The weight ratio of oil phase to aqueous phase in the emulsion can generally be between 40:1 and 1:50, although the higher proportions of aqueous phase are economically disadvantageous particularly when forming a suspension of microcapsules. Usually the weight ratio of oil phase to aqueous phase is between 2:1 and 1:3. If the oil phase composition is highly viscous, a phase inversion process can be used in which the oil phase is mixed with surfactant and a small amount of water, for example 2.5 to 10% by weight based on the oil phase, forming a water-in-oil emulsion which inverts to an oil-in-water emulsion as it is sheared. Further water can then be added to dilute the emulsion to the required concentration.

The second and third steps of the present process involve adding a water reactive silicon compound comprising tetraalkoxysilane to the oil in water emulsion, and polymerizing the tetraalkoxysilane at the oil/water interface of the emulsion. Although not wishing to be bound by any theory, the present inventors believe the third step of the effects an "ex-situ emulsion polymerization" by which the tetraalkoxysilane precursor hydrolyzes and condenses at the oil/water interface leading to the formation of core-shell microcapsules via a phase transfer of the precursors.

The tetraalkoxysilane, such as tetraethoxysilane (TEOS), can be used in monomeric form or as a liquid partial condensate. The tetraalkoxysilane can be used in conjunction with one or more other water-reactive silicon compound having at least two, preferably at least 3, Si-OH groups or hydrolysable groups bonded to silicon, for example an alkyltrialkoxysilane such as methyltrimethoxysilane or a liquid condensate of an alkyltrialkoxysilane. Hydrolysable groups can for example be alkoxy or acyloxy groups bonded to silicon. The water reactive silicon compound can for example comprise 75-100% by weight tetraalkoxysilane and 0-25% trialkoxysilane. The alkyl and alkoxy groups in the tetraalkoxysilanes or other silanes preferably contain 1 to 4 carbon atoms, most preferably 1 or 2 carbon atoms. The tetraalkoxysilane, and other water-reactive silicon compound if used, hydrolyses and condenses to form a network polymer, that is a 3-dimensional network of silicon-based material, around the emulsified droplets of the lipophilic active material composition. The water-reactive silicon compound preferably consists of at least 75%, and most preferably 90-100% tetraalkoxysilane. We have found that a tetraalkoxysilane effectively forms impermeable microcapsules, forming a 3-dimensional network consisting substantially of $SiO_{4/2}$ units.

The tetraalkoxysilane, and other water reactive silicon compounds if used, can be added to the emulsion of active material composition as an undiluted liquid or as a solution in an organic solvent or in an emulsion form. The tetraalkoxysilane and the oil in water emulsion are mixed during addition and subsequent polymerization to form the silicon-based polymer shell on the surface of the emulsified droplets. Mixing is typically effected with stirring techniques. Common stirring techniques are typically sufficient to maintain the particle size of the starting oil in water emulsion while allowing the tetraalkoxysilane to polymerize and condense at the oil water interface

The amount of tetraalkoxysilane added in step II typically ranges from 6/1 to 1/13, alternatively from 1.2/1 to 1/7.3, alternatively from 1.3 to 1/6.1 based on the weight amount of oil phase present in the emulsion.

The polymerization of the tetraalkoxysilane at the oil/water interface typically is a condensation reaction which may be conducted at acidic, neutral or basic pH. The condensation reaction is generally carried out at ambient temperature and pressure, but can be carried out at increased temperature, for example up to 95°C, and increased or decreased pressure, for example under vacuum to strip the volatile alcohol produced during the condensation reaction.

Any catalyst known to promote the polymerization of the tetraalkoxysilane may be added to step III to form the shell of the microcapsule. The catalyst is preferably an oil soluble organic metal compound, for example an organic tin compound, particularly an organotin compound such as a diorganotin diester, for example dimethyl tin di(neodecanoate), dibutyl tin dilaurate or dibutyl tin diacetate, or alternatively a tin carboxylate such as stannous octoate, or an organic titanium compound such as tetrabutyl titanate. An organotin catalyst can for example be used at 0.05 to 2% by weight based on the water reactive silicon compound. An organotin catalyst has the advantage of effective catalysis at neutral pH. The catalyst is typically mixed with the oil phase components before it is emulsified, since this promotes condensation of the water reactive silicon compound at the surface of the emulsified oil phase droplets. A catalyst can alternatively be added to the emulsion before the addition of the water-reactive silicon compound, or simultaneously with the tetraalkoxysilane, or after the addition of the tetraalkoxysilane to harden and make more impervious the shell of silicon-based polymer which has been formed. Encapsulation can however be achieved without catalyst. The catalyst, when used, can be added undiluted, or as a solution in an organic solvent such as a hydrocarbon, alcohol or ketone, or as a mutiphasic system such as an emulsion or suspension.

In one embodiment, the polymerization reaction in step III) is allowed to proceed so as to form the shell of a microcapsule that is at least 18 nanometers thick, alternatively the shell has a thickness in the range of 18 to 150 nanometers, alternatively from 18 to 100 nanometers.

Shell thicknesses may be determined from the particle size (PS) of the resulting microcapsules in suspension and the amounts of the oil phase and tetraalkoxysilane used in the process to prepare them according to the following:

$$\text{Shell Thickness (nm)} = [(PS/2)-[(PS/2)*(Payload/100)^{1/3})]*1000$$

where PS is particle size (Dv 0.5) expressed in micrometers payload = Volume oil phase *100/(Volume oil phase+Volume shell) Volume oil phase = Mass oil phase/density of oil phase Volume shell = Mass shell/density of the shell

This equation is based on the spherically shaped microcapsules having an average diameter as determined by their average particle size (Dv 0.5). Thus, the shell thickness is the difference between the radius of the microcapsule and the radius of the core material in the microcapsule.

$$\text{Shell thickness} = r_{microcapsule} - r_{core}$$

where

$$r_{microcapsule} = (PS)/2$$

and

$$r_{core} = (PS/2)*(Payload/100)^{1/3})$$

Payload represents the percentage of the microcapsule occupied by the core material, as determined by the amount of oil phase present in the emulsion. Thus, payload is calculated by the relationship;

$$\text{Payload} = \text{Volume oil phase}*100/(\text{Volume oil phase} + \text{Volume shell})$$

The volume oil phase = mass oil phase/density of oil phase. The mass of the oil phase in this equation is the same as the amount used in the process (as per step I) to prepare the microcapsules. In one embodiment of the present invention, the oil phase is ethylhexy methoxycinnamate (EHMC) having a density of 1.011 g/mL.

The volume of the shell = mass of shell / density of silica. The silicon based polymer comprising the shell is expected to have an average chemical composition with the empirical formula $SiO_2$. Thus, the density of the shell is estimated to be 2 g/mL, which approximates the density of silica ($SiO_2$). The mass of the shell is calculated from the amount of tetraalkoxysilane added to the process (as per step II). More specifically, the mass of the shell is based on the expected stoichiometric yield of silicon based polymer of empirical formula $Si0_2$ given the type and amount of the tetraalkoxysilane used in the process. In one embodiment, the tetraalkoxysilane is tetraethoxysilane (TEOS) having a density of 0.934 g/mL. In this embodiment, the assumed complete hydrolysis and condensation of 1 g of TEOS produces 0.288 g of $SiO_2$ polymer (silica).

## Examples

These examples are intended to illustrate the invention to one of ordinary skill in the art and should not be interpreted as limiting the scope of the invention set forth in the claims. All measurements and experiments were conducted at 23°C, unless indicated otherwise. All % refer to weight percent, unless indicated otherwise.

<u>Test Methods</u>

Volume fraction - was determined by summing the volume of each component added. The volume was calculated by dividing the mass by density.

Solid content - was determined by placing 2.5 g of the continuous phase of the suspension in an oven at 170°C and monitoring the sample until a constant weight was achieved.

Example 1 (comparative)

350 g EHMC (Parsol MCX®) was emulsified in 540.9 g water containing 1.4 g Pareth-3 nonionic polyethylene glycol lauryl ether surfactant and 0.9 g cetyl trimethyl ammonium chloride (CTAC) cationic surfactant. The coarse emulsion was passed once through a "APV Model 1000" homogeniser operating at 50 bars. 12 % tetraethoxysilane (TEOS) was added to the emulsion while stirring to form a coarse emulsion of microcapsules. Microcapsules of average volume particle size (Dv 0.5) 3.23 micrometers (μm) were produced in suspension. The solid content of the continuous phase obtained by filtration of the lipophilic liquid core (EHMC) / silicate (silica) shell microcapsules suspension was 2.71 %. The microcapsule suspension gelled after one day at 50°C.

Example 2

An identical microcapsule suspension was prepared as in Example 1. However, the suspension was ultrafiltered with parallel addition of water. The solid content of the resulting suspension was reduced to 0.29%. The corresponding microcapsule suspension was stable for more than one month at 50°C without gelation.

Example 3 (comparative)

350 g EHMC (Parsol MCX®) was emulsified in 540.9g water containing 1.4g Pareth-3 nonionic polyethylene glycol lauryl ether surfactant and 0.9g cetyl trimethyl ammonium chloride (CTAC) cationic surfactant. The coarse emulsion was passed once through a "APV Model 1000" homogeniser operating at 50 bars. 12 % TEOS was added to the emulsion while stirring to form a coarse emulsion of microcapsules. Microcapsules of average volume particle size (Dv 0.5) 3.23 micrometers (um) were produced in suspension. The initial solid content of the continuous phase obtained by filtration of the lipophilic liquid core (EHMC) / silicate (silica) shell microcapsules suspension was 2.71 %. The subsequent filtration of the suspension without parallel addition of water to the suspension allowed to reach a microcapsule volume fraction of 54.8 %. The highly concentrated suspension gels after one day at 50°C.

Example 4

An identical microcapsule suspension was prepared as in Example 3. However, the suspension was filtered with parallel addition of water. The solid content of the resulting suspension was reduced to 0.29%. Then the suspension was concentrated by ultrafiltration to reach a microcapsule volume fraction of 54.8 %.
The corresponding microcapsule suspension withstands more than one month at 50°C without gelation.

Example 5 (comparative)

250 g EHMC (Parsol MCX®) was emulsified in 172.5g water containing 0.7g Pareth-3 nonionic polyethylene glycol lauryl ether surfactant and 0.46g cetyl trimethyl ammonium chloride (CTAC) cationic surfactant. The coarse emulsion was passed once through an "APV Model 1000" homogeniser operating at 50 bars. 17.14% TEOS was added to the emulsion while stirring to form a coarse emulsion of microcapsules. Microcapsules of average volume particle size (Dv 0.5) 3.35 micrometers (um) were produced in suspension. The suspension microcapsule volume fraction is 55%.The initial solid content of the continuous phase obtained by filtration of the lipophilic liquid core (EHMC) / silicate (silica) shell microcapsules suspension was 3.56%. The highly concentrated suspension gels after one day at 50°C.

Example 6

An similar microcapsule suspension was prepared as in Example 5, but having an average volume particle size (Dv 0.5) 3.43 micrometers (μm) and a volume fraction of 55%. The initial solid content of the continuous phase of the lipophilic liquid core (EHMC) / silicate (silica) shell microcapsules suspension was 3.56%. The subsequent ultrafiltration of the suspension with parallel addition of water to the suspension reduced the solid content of the continuous phase to 0.11 %. No gelation of the highly concentrated suspension was observed during the filtration process. The resulting microcapsule suspension was stable for more than one month at 50°C without gelation.

Example 7 (comparative)

250 g EHMC (Parsol MCXO) was emulsified in 172.5g water containing 0.7g Pareth-3 nonionic polyethylene glycol lauryl ether surfactant and 0.46g cetyl trimethyl ammonium chloride (CTAC) cationic surfactant. The coarse emulsion was passed once through an "APV Model 1000" homogeniser operating at 50 bars. 17.14% TEOS was added

to the emulsion while stirring to form a coarse emulsion of microcapsules. Microcapsules of average volume particle size (Dv 0.5) 3.43 micrometers (μm) were produced in suspension. The suspension microcapsule volume fraction was 52%.The initial solid content of the continuous phase obtained by filtration of the lipophilic liquid core (EHMC) / silicate (silica) shell microcapsules suspension was 3.92%. The neutralization to pH 8 of the suspension by NaOH 0.1 M without parallel addition of water to the suspension led to quick gelation of the concentrated suspension.

Example 8

An identical microcapsule suspension was prepared as in Example 7. However, the suspension was ultrafiltered with parallel addition of water. The solid content of the resulting suspension was reduced to 0.15%. The suspension microcapsule volume fraction was 52%. The neutralization to pH 8 of the suspension by NaOH 0.1 M without parallel addition of water to the suspension did not lead to the gelation of the concentrated suspension. The corresponding microcapsule suspension was stable for more than one month at 50°C without gelation.

Example 9 (comparative)

175 g EHMC (Parsol MCX®) was emulsified in 270.5g water containing 0.7g Pareth-3 nonionic polyethylene glycol lauryl ether surfactant and 0.46g cetyl trimethyl ammonium chloride (CTAC) cationic surfactant. The coarse emulsion was passed once through an "APV Model 1000" homogeniser operating at 750 bars. 35% TEOS was added to the emulsion while stirring to form a coarse emulsion of microcapsules. Microcapsules of average volume particle size (Dv 0.5) 1.97 micrometers (um) were produced in suspension. The suspension microcapsule volume fraction was 36%.The initial solid content of the continuous phase obtained by filtration of the lipophilic liquid core (EHMC) / silicate (silica) shell microcapsules suspension was 6.09%. The neutralization to pH 8 of the suspension by NaOH 0.1 M without parallel addition of water to the suspension leads to quick gelation of the concentrated suspension. The suspension gels after one day at 50°C.

Example 10

An identical microcapsule suspension was prepared as in Example 9. However, the suspension was ultrafiltered with parallel addition of water. The solid content of the resulting suspension was reduced to 0.08%. The calculated shell thickness was 31 nm. The corresponding microcapsule suspension was stable for more than one month at 50°C without gelation.

Example 11 (comparative)

242,04g EHMC (Parsol MCX®) was mixed with 73.8g TEOS. The organic phase was emulsified in 155.45g of aqueous solution with 0.62g of cetyltrimethyl ammonium chlorite (CTAC) under high shear forces using a mixer IKA Ultra-Turax T 25 Basic at 9600 rpm during 5 minutes .This emulsion was poured in a reactor containing 269.66g of an aqueous solution having a pH of 2.5. The mixture was stirred at 400 rpm until the emulsion was completely mixed, then the stirring was lowered to 60 rpm during 24 hours. Microcapsules of average volume particle size (Dv0,5) = 3.6 μm were produced in suspension. The suspension microcapsules volume fraction = 35%. The initial solid content of the continuous phase obtained by filtration of the lipophilic liquid core shell microcapsules suspension was 3.07 %. This microcapsule suspension gelled after 1 day at 50°C.

Example 12 (comparative)

The suspension of example 11 was subjected to ultrafiltration without parallel addition of water allowed which increased the volume fraction of the suspension to 55%. The corresponding microcapsule suspension gelled after 1 day at 50°C.

Example 13

The suspension of example 11 was subjected to ultrafiltration with parallel addition of water which reduced the solid content of the continuous phase to 0.03%. The microcapsule suspension was stable for more than 6 days at 50°C without gelation.

Example 14 (comparative)

242,04 g EHMC (Parsol MCX®) was mixed with 73.8g TEOS. The organic phase was emulsified in 155.45g of aqueous solution with 0.62g of cetyltrimethyl ammonium chlorite (CTAC) under high shear forces using a mixer IKA Ultra-Turax T 25 Basic at 9600 rpm during 5 minutes .This emulsion was poured in a reactor containing 269.66g of an aqueous solution having a pH of 2.5. The mixture was stirred at 400 rpm until the emulsion was completely mixed, then the stirring was lowered to 60 rpm during 24 hours. Microcapsules of average volume particle size (DV0,5) = 3.6 μm were produced in suspension. The suspension microcapsules volume fraction = 35 %. The initial solid content of the continuous phase obtained by filtration of the lipophilic liquid core shell microcapsules suspension was 3.07 %. The subsequent filtration of the suspension with parallel addition of water to the suspension allowed reducing the solid content of the continuous phase down to 0.03%.After that, this suspension was concentrated filtering without parallel addition of water to have a solid content to 55%. The corresponding microcapsule suspension gelled after 1 day at 50°C.

Example 15 (comparative)

250g EHMC (Parsol MCX®) was mixed with 63.19g TEOS. The organic phase was emulsified in 63.1g of aqueous solution with 0.45g of cetyltrimethyl ammonium chlorite (CTAC) under high shear forces using a mixer IKA Ultra-Turax T 25 Basic at 9600 rpm during 5 minutes .This emulsion was poured in a reactor containing 109.45g of an aqueous solution having a pH of 2.5. The mixture was stirred at 400 rpm until the emulsion was completely mixed, then the stirring was lowered to 60 rpm during 24 hours. Microcapsules of average volume particle size (Dv0,5) = 4.2 μm were produced in suspension. The suspension microcapsules volume fraction = 50%. The initial solid content of the continuous phase obtained by filtration of the lipophilic liquid core shell microcapsules suspension was 5.41 %. This microcapsule suspension gelled after 1 day at 50°C.

Example 16 (comparative)

The microcapsule suspension of Example 15 was neutralized by adding sodium hydroxide (0.1 M) to a pH of 7.5-8.5. The suspension gelled after 1 day at 50°C.

Example 17

The suspension of example 15 was subjected to ultrafiltration with parallel addition of water which reduced the solid content of the continuous phase to 0.16%. The corresponding microcapsule suspension was stable for more than 6 days at 50°C without gelation.

Example 18

The suspension of Example 15 was subjected to ultrafiltration with parallel addition of water which reduced the solid content of the continuous phase to 0.16%. The suspension was neutralized by adding sodium hydroxide (0.1 M) to a pH of 7.5-8.5. The corresponding microcapsule suspension was stable for more than 6 days at 50°C without gelation.

Example 19 (comparative)

350.0 g. EHMC (Parsol MCX®) was emulsified in 540.9 g. water containing 1.3 g. Pareth-3 nonionic polyethylene glycol lauryl ether surfactant and 3.2 g. cetyl trimethyl ammonium chloride (CTAC) cationic surfactant. The coarse emulsion was passed once through an "APV Model 2000" homogeniser operating at 100 bars. 12 % TEOS was added to the emulsion while stirring to form a coarse emulsion of microcapsules. Microcapsules of average volume particle size (Dv 0.5) 1.99 micrometers (μm) were produced in suspension. The solid content of the continuous phase obtained by filtration of the lipophilic liquid core (EHMC) / silicate (silica) shell microcapsules suspension was 2.17%. The microcapsule suspension gelled after one day at 50°C.

| | Dv 0 5 (μm) | Suspension Volume Fraction | TEOS Level (%) | Solid Content in Continuous phase (%) | Neutralization at pH 8 | Gel Time at 50°C (Days) |
|---|---|---|---|---|---|---|
| Example 1 | 323 | 34 8 | 12 | 271 | No | 1 |
| Example 2 | 323 | 34 8 | 12 | 029 | No | > 30 |

(continued)

| | Dv 0 5 (μm) | Suspension Volume Fraction | TEOS Level (%) | Solid Content in Continuous phase (%) | Neutralization at pH 8 | Gel Time at 50°C (Days) |
|---|---|---|---|---|---|---|
| Example 3 | 323 | 54 8 | 12 | 271 | No | 1 |
| Example 4 | 323 | 54 8 | 12 | 029 | No | > 30 |
| Example 5 | 335 | 55 | 17 14 | 3 56 | No | 1 |
| Example 6 | 343 | 55 | 17 14 | 3 56 | No | > 30 |
| Example 7 | 343 | 52 | 17 14 | 3 92 | Yes | 0 |
| Example 8 | 343 | 52 | 17 14 | 0 15 | Yes | > 30 |
| Example 9 | 1 97 | 36 | 35 | 609 | Yes | 0 |
| Example 9 | 1 97 | 36 | 35 | 609 | No | 1 |
| Example 10 | 1 97 | 36 | 35 | 008 | No | > 30 |
| Example 11 | 36 | 35 | 126 | 307 | No | 1 |
| Example 12 | 36 | 55 | 12 6 | 307 | No | 1 |
| Example 13 | 3 6 | 35 | 126 | 003 | No | 6 |
| Example 14 | 36 | 55 | 126 | 307 | No | 1 |
| Example 15 | 42 | 50 | 13 | 541 | No | 1 |
| Example 16 | 42 | 50 | 13 | 541 | Yes | 1 |
| Example 17 | 42 | 50 | 13 | 0 16 | No | 6 |
| Example 18 | 4 2 | 50 | 13 | 0 16 | Yes | 6 |
| Example 19 | 1 99 | 50 | 13 | 0 16 | Yes | 6 |

Example 20 (Comparative)

175.0 g. EHMC (Parsol MCX®) was emulsified in 270.45 g. water containing 0.65 g. Pareth-3 nonionic polyethylene glycol lauryl ether surfactant and 1.6 g. cetyl trimethyl ammonium chloride (CTAC) cationic surfactant. The coarse emulsion was passed once through an "APV Model 2000" homogeniser operating at 100 bars. 12 % TEOS was added to the emulsion while stirring to form a coarse emulsion of microcapsules. Microcapsules of average volume particle size (Dv 0.5) 1.99 micrometers (um) were produced in suspension. The solid content of the continuous phase obtained by filtration of the lipophilic liquid core (EHMC) / silicate (silica) shell microcapsules suspension was 2.17%. 2% of a 50% solution of a silicone polyether (Dow Corning ® 2-5657) was post added to the suspension. The corresponding microcapsule suspension was stable for more than one month at 50°C without gelation.

Example 21 (Comparative)

2% of a 50% solution of a silicone polyether (Dow Corning ® 2-5657) was post added to the suspension of Example 19. The corresponding microcapsule suspension was stable for more than one month at 50°C without gelation.

Example 22 (Comparative)

175.0 g. EHMC (Parsol MCX®) was emulsified in 270.45 g. water containing 0.65 g. Pareth-3 nonionic polyethylene glycol lauryl ether surfactant and 1.6 g. cetyl trimethyl ammonium chloride (CTAC) cationic surfactant. The coarse emulsion was passed once through an "APV Model 2000" homogeniser operating at 50 bars. 12 % TEOS was added to the emulsion while stirring to form a coarse emulsion of microcapsules. Microcapsules of average volume particle size (Dv 0.5) 3.01 micrometers (um) were produced in suspension. The solid content of the continuous phase obtained by filtration of the lipophilic liquid core (EHMC) / silicate (silica) shell microcapsules suspension was 3.04%. 2% of a 50% solution of a silicone polyether (EO=12) was post added to the suspension, along with 0.1% Methocel

K100M (as 10% slurry in propylene glycol). The corresponding microcapsule suspension withstands more than one month at 50°C without gelation, but did show settling and separation instability.

Example 23 (Comparative)

175.0 g. EHMC (Parsol MCX®) was emulsified in 270.45 g. water containing 0.65 g. Pareth-3 nonionic polyethylene glycol lauryl ether surfactant and 1.6 g. cetyl trimethyl ammonium chloride (CTAC) cationic surfactant. The coarse emulsion was passed once through an "APV Model 2000" homogeniser operating at 50 bars. 12 % TEOS was added to the emulsion while stirring to form a coarse emulsion of microcapsules. Microcapsules of average volume particle size (Dv 0.5) 3.01 micrometers (um) were produced in suspension. The solid content of the continuous phase obtained by filtration of the lipophilic liquid core (EHMC) / silicate (silica) shell microcapsules suspension was 3.04%. 2% of a 50% solution of a silicone polyether (EO=12) was post added to the suspension, along with 0.4% Methocel K100M (as 10% slurry in propylene glycol). The corresponding microcapsule suspension withstands more than one month at 50°C without gelation or settling and separation instability.

Example 24 (Comparative)

280 g. Delta-Damascone was emulsified in 515.02 g water containing 1.6 g Pareth-3 nonionic polyethylene glycol lauryl ether surfactant and 0.93 g. cetyl trimethyl ammonium chloride (CTAC) cationic surfactant. The coarse emulsion was mixed in an IKA Ultra-Turax T 25 Basic at 24000 rpm during 180 seconds. 10 % TEOS was added to the emulsion while stirring to form after its hydrolysis and condensation a suspension of microcapsules. Microcapsules of average volume particle size (Dv 0.5) 3.2 micrometers (um) were produced in suspension. The microcapsule content was 34.8 % w/w of the suspension. The solid content of the continuous phase obtained by filtration of the lipophilic liquid core (D-Damascone) / silicate (silica) shell microcapsules suspension was 2.7 %. The corresponding microcapsule suspension withstands not more than three days at 50°C without gelation.

Example 25 (Comparative)

0.32 g of nOctyltriethoxysilane (Dow Corning ® Z-6341) has been added to 19.7 g of suspension obtained in example 24 under mixing. The silane /microcapsule ratio is 4.6 % w/w. After one day RT the suspension has been placed in an oven at 50°C. The corresponding microcapsule suspension withstands not more than seven days at 50°C without gelation.

Example 26 (Comparative)

1.26 g of nOctyltriethoxysilane (Dow Corning ® Z-6341) has been added to 20 g of the suspension obtained in example 24 under mixing. The silane / microcapsule ratio is 18 % w/w. After one day RT the suspension has been placed in an oven at 50°C. The corresponding microcapsule suspension withstands not more than 14 days at 50°C without gelation.

Example 27 (Comparative)

0.16 g of isoButyltriethoxysilane (Dow Corning ® Z-6403) has been added to 20 g of the suspension obtained in example #24 under mixing. The silane / microcapsule ratio is 2.3 % w/w. After one day RT the suspension has been placed in an oven at 50°C. The corresponding microcapsule suspension withstands not more than 11 days at 50°C without gelation.

Example 28 (Comparative)

0.32 g of isoButyltriethoxysilane (Dow Corning ® Z-6403) has been added to 20 g of the suspension obtained in example #24 under mixing. The silane / microcapsule ratio is 4.6 % w/w. After one day RT the suspension has been placed in an oven at 50°C. The corresponding microcapsule suspension withstands not more than 5 days at 50°C without gelation.

Example 29 (Comparative)

0.08 g of Cetrimoniumpropyltrimethoxysilane Chloride (Dow Corning ® Q9-6346) has been added to 20 g of the suspension obtained in example #24 under mixing. The silane / microcapsule ratio is 1.1 % w/w. After one day RT

the suspension has been placed in an oven at 50°C. The corresponding microcapsule suspension withstands more than one months at 50°C without gelation.

Example 30 (Comparative)

0.20 g of Cetrimoniumpropyltrimethoxysilane Chloride (Dow Corning ® Q9-6346) has been added to 20 g of the suspension obtained in example #24 under mixing. The silane / microcapsule ratio is 2.9 % w/w. After one day RT the suspension has been placed in an oven at 50°C. The corresponding microcapsule suspension withstands more than 50 days at 50°C without gelation.

Example 31 (Comparative)

0.06 g of GlycidyltrimethylamoniumChloride PDMS (Dow Corning ® 7-6030) has been added to 20 g of the suspension obtained in example #24 under mixing. The silane / microcapsule ratio is 0.9 % w/w. After one day RT the suspension has been placed in an oven at 50°C. The corresponding microcapsule suspension withstands not more than 7 days at 50°C without gelation.

Example 32 (Comparative)

0.16 g of GlycidyltrimethylamoniumChloride PDMS (Dow Corning ® 7-6030) has been added to 20 g of the suspension obtained in example #24 under mixing. The silane / microcapsule ratio is 2.3 % w/w. After one day RT the suspension has been placed in an oven at 50°C. The corresponding microcapsule suspension withstands not more than 7 days at 50°C without gelation

Example 33 (Comparative)

0.06 g of trihydroxysilylpropylmethylphosphonate (Dow Corning ® Q1-6083) has been added to 20 g of the suspension obtained in example #24 under mixing. The silane / microcapsule ratio is 0.9 % w/w. After one day RT the suspension has been placed in an oven at 50°C. The corresponding microcapsule suspension withstands not more than 1 days at 50°C without gelation

Example 34 (Comparative)

0.32 g of trihydroxysilylpropylmethylphosphonate (Dow Corning ® Q1-6083) has been added to 20 g of the suspension obtained in example #24 under mixing. The silane / microcapsule ratio is 4.6 % w/w. After one day RT the suspension has been placed in an oven at 50°C. The corresponding microcapsule suspension withstands not more than 1 days at 50°C without gelation

| | Dv0.5 (μm) | Suspension Solid Content (%) | TEOS Level (%) | Solid Content in Continuous phase (%) | Neutralisation at pH 8 | Post added Silane | w/w% | Gel Time at 50°C (Days) |
|---|---|---|---|---|---|---|---|---|
| Example 24 | 3.2 | 34.8 | 10 | 2.7 | No | None | 0 | 3 |
| Example 25 | 3.2 | 34.8 | 10 | 2.7 | No | Z-6341 | 4.6 | 10 |
| Example 26 | 3.2 | 34.8 | 10 | 2.7 | No | Z-6341 | 18 | 14 |
| Example 27 | 3.2 | 34.8 | 10 | 2.7 | No | Z-6403 | 2.3 | 11 |
| Example 28 | 3.2 | 34.8 | 10 | 2.7 | No | Z-6403 | 4.6 | 5 |
| Example 29 | 3.2 | 34.8 | 10 | 2.7 | No | Z-6346 | 1.1 | >50 |

(continued)

| | Dv0.5 (μm) | Suspension Solid Content (%) | TEOS Level (%) | Solid Content in Continuous phase (%) | Neutralisation at pH 8 | Post added Silane | w/w% | Gel Time at 50°C (Days) |
|---|---|---|---|---|---|---|---|---|
| Example 30 | 3.2 | 34.8 | 10 | 2.7 | No | Z-6346 | 2.9 | >50 |
| Example 31 | 3.2 | 34.8 | 10 | 2.7 | No | 7-6030 | 0.9 | 7 |
| Example 32 | 3.2 | 34.8 | 10 | 2.7 | No | 7-6030 | 2.3 | 7 |
| Example 33 | 3.2 | 34.8 | 10 | 2.7 | No | Q1-6083 | 0.9 | 1 |
| Example 34 | 3.2 | 34.8 | 10 | 2.7 | No | Q1-6083 | 4.6 | 1 |

The present application is a divisional application based on an earlier European Application No 09792407.0, which was in turn derived from PCT Application No PCT/US2009/056466. The following numbered clauses, which correspond to the claims of that earlier PCT Application as filed, form part of the present disclosure but do not form part of the claimed invention.

Clauses

1. An aqueous suspension of silicate shell microcapsules having a volume fraction of microcapsules of at least 30 % and is gel free at 50°C for at least one month.

2. The suspension may have a non-volatile solid content of less than 0.3 weight percent.

3. The suspension may contain less than 0.3 weight percent colloidal silicate particles.

4. The suspension may further comprise a colloidal silicate sequestering agent.

5. The colloidal silicate sequestering agent may be an organofunctional silane.

6. The organofunctional silane may be a quat functional trialkoxysilane.

7. The organofunctional silane may be cetrimoniumpropyltrimethoxysilane chloride.

8. The colloidal silicate sequestering agent may be a silicone polyether.

9. The silicone polyether has the average formula

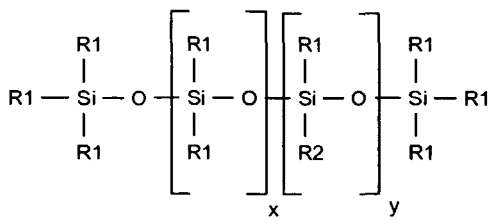

or

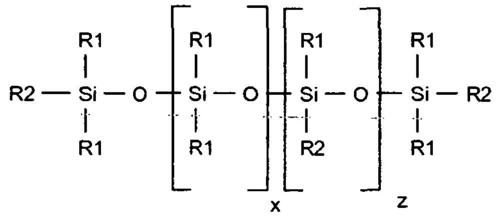

R1 is an alkyl group containing 1-6 carbon atoms;
R2 is a polyether group having the formula

$$-(CH_2)_aO(C_2H_4O)_b(C_3H_6O)_cR3;$$

x has a value of 10-1,000,
y has a value of 2-500,
z has a value of 2-500,
a has a value of 3-6; b has a value of 4-30; c has a value of 0-30; and R3 is hydrogen, a methyl radical, or an acyl group.

10. The values of x, y and z may be as follows:

x has a value of 20 - 100,
y has a value of 2-10,
z has a value of 2-10, in the silicone polyether formula.

## Claims

1. A process for improving the stability of an aqueous suspension of silicate shell microcapsules comprising reducing non-volatile solid content of the aqueous suspension of the silicate shell microcapsules to less than 0.3 weight percent.

2. The process of claim 1 where the non volatile content of the aqueous suspension of the silicate shell microcapsules is reduced by removing colloidal silicate particles.

3. The process of claim 2 wherein at least 50 weight % of the colloidal silicate particles are removed from the aqueous suspension of the silicate shell microcapsules.

4. The process of claim 2 wherein the colloidal silicate particles are removed from the aqueous suspension of the silicate shell microcapsules by ultrafiltration.

5. The process of claim 4 wherein the ultrafiltration occurs with parallel addition of water to the suspension of the silicate shell microcapsules.

6. The process of claim 5 wherein the amount of water added to the suspension is 90 to 110% of the amount of water removed as permeate during ultrafiltration.

7. The process of any one of claims 1- 6 wherein the silicate shell microcapsules are obtained by;

I) mixing an oil phase and an aqueous solution of a cationic surfactant to form an oil in water emulsion,
II) adding a water reactive silicon compound comprising a tetraalkoxysilane to the oil in water emulsion,
III) polymerizing the tetraalkoxysilane at the oil/water interface of the emulsion to form a microcapsule having a core containing the oil and a silicate shell.

8. The process of claim 7 wherein the tetraalkoxysilane is tetraethoxysilane.

9. The process of claim 8 wherein the weight % of cationic surfactant to the oil phase in the emulsion of step I) ranges

from 0.1% to 0.3% and the shell thickness of the microcapsule is at least 18 nanometers.

**10.** The process of claim 7 wherein the oil phase comprises a sunscreen.

**Patentansprüche**

**1.** Ein Verfahren zur Verbesserung der Stabilität einer wässrigen Suspension von Mikrokapseln mit Silikathülle, beinhaltend das Reduzieren des nichtflüchtigen Feststoffgehalts der wässrigen Suspension der Mikrokapseln mit Silikathülle auf weniger als 0,3 Gewichtsprozent.

**2.** Verfahren gemäß Anspruch 1, wobei der nichtflüchtige Gehalt der wässrigen Suspension der Mikrokapseln mit Silikathülle durch Entfernen kolloidaler Silikatpartikel reduziert wird.

**3.** Verfahren gemäß Anspruch 2, wobei mindestens 50 Gew.-% der kolloidalen Silikatpartikel aus der wässrigen Suspension der Mikrokapseln mit Silikathülle entfernt werden.

**4.** Verfahren gemäß Anspruch 2, wobei die kolloidalen Silikatpartikel durch Ultrafiltration aus der wässrigen Suspension der Mikrokapseln mit Silikathülle entfernt werden.

**5.** Verfahren gemäß Anspruch 4, wobei die Ultrafiltration mit paralleler Zugabe von Wasser zu der Suspension der Mikrokapseln mit Silikathülle erfolgt.

**6.** Verfahren gemäß Anspruch 5, wobei die Menge an Wasser, das zu der Suspension zugegeben wird, 90 bis 110 % der Menge an Wasser, das während der Ultrafiltration als Permeat entfernt wird, beträgt.

**7.** Verfahren gemäß einem der Ansprüche 1-6, wobei die Mikrokapseln mit Silikathülle folgendermaßen erhalten werden:

I) Mischen einer Ölphase und einer wässrigen Lösung eines kationischen Tensids, um eine Öl-in-Wasser-Emulsion zu bilden,
II) Zugeben einer mit Wasser reaktiven Siliziumverbindung, beinhaltend ein Tetraalkoxysilan, zu der Öl-in-Wasser-Emulsion,
III) Polymerisieren des Tetraalkoxysilans an der Öl/Wasser-Grenzfläche der Emulsion, um eine Mikrokapsel mit einem Kern, der das Öl enthält, und einer Silikathülle zu bilden.

**8.** Verfahren gemäß Anspruch 7, wobei das Tetraalkoxysilan Tetraethoxysilan ist.

**9.** Verfahren gemäß Anspruch 8, wobei das Gew.-% des kationischen Tensids zu der Ölphase in der Emulsion aus Schritt I) im Bereich von 0,1 % bis 0,3 % liegt und die Hüllendicke der Mikrokapsel mindestens 18 Nanometer beträgt.

**10.** Verfahren gemäß Anspruch 7, wobei die Ölphase ein Sonnenschutzmittel beinhaltet.

**Revendications**

**1.** Un procédé pour améliorer la stabilité d'une suspension aqueuse de microcapsules à coques de silicate comprenant la réduction de la teneur en solides non volatils de la suspension aqueuse des microcapsules à coques de silicate à moins de 0,3 pour cent en poids.

**2.** Le procédé de la revendication 1 où la teneur en matière non volatile de la suspension aqueuse des microcapsules à coques de silicate est réduite en enlevant des particules de silicate colloïdal.

**3.** Le procédé de la revendication 2 dans lequel au moins 50 % en poids des particules de silicate colloïdal sont enlevés de la suspension aqueuse des microcapsules à coques de silicate.

**4.** Le procédé de la revendication 2 dans lequel les particules de silicate colloïdal sont enlevées de la suspension aqueuse des microcapsules à coques de silicate par ultrafiltration.

5. Le procédé de la revendication 4 dans lequel l'ultrafiltration se produit avec ajout en parallèle d'eau dans la suspension des microcapsules à coques de silicate.

6. Le procédé de la revendication 5 dans lequel la quantité d'eau ajoutée dans la suspension représente 90 à 110 % de la quantité d'eau enlevée en tant que perméat durant l'ultrafiltration.

7. Le procédé de l'une quelconque des revendications 1 à 6 dans lequel les microcapsules à coques de silicate sont obtenues :

   I) en mélangeant une phase huileuse et une solution aqueuse d'un tensioactif cationique pour former une émulsion huile dans l'eau,
   II) en ajoutant un composé de silicium réactif dans l'eau comprenant un tétraalkoxysilane dans l'émulsion huile dans l'eau,
   III) en polymérisant le tétraalkoxysilane à l'interface huile/eau de l'émulsion pour former une microcapsule ayant un coeur contenant l'huile et une coque de silicate.

8. Le procédé de la revendication 7 dans lequel le tétraalkoxysilane est du tétraéthoxysilane.

9. Le procédé de la revendication 8 dans lequel le % en poids du tensioactif cationique rapporté à la phase huileuse dans l'émulsion de l'étape I) est compris dans la gamme allant de 0,1 % à 0,3 % et l'épaisseur de coque de la microcapsule est d'au moins 18 nanomètres.

10. Le procédé de la revendication 7 dans lequel la phase huileuse comprend un écran solaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61096397 B
- EP 0934773 A
- EP 0941761 A
- US 6303149 B
- WO 03066209 A
- GB 2416524 A
- WO 2005009604 A1
- US 6159453 A
- US 6238650 B
- WO 2005009604 A
- US 5035832 A